# EUROPEAN PATENT SPECIFICATION

(11) **EP 0 640 357 B1**
(45) Date of publication and mention of the grant of the patent: **24.11.1999**
(21) Application number: 94108490.7
(22) Date of filing: 01.06.1994
(51) Int. Cl.: A61M 16/12

(54) **Gas mixture and device for delivering the gas mixture to the lungs of a living being**
Gasmischung und Vorrichtung zur Zuführung der Gasmischung zu den Lungen eines Lebewesens
Mélange de gaz et dispositif de fourniture du mélange gaseux aux poumons d'un être vivant

(30) Priority: 22.07.1993 SE 9302478
(43) Date of publication of application: 01.03.1995
(73) Proprietor: Siemens-Elema AB, 171 95 Solna 1 (SE)
(72) Inventor: Olsson, Sven-Gunnar, S-232 00 Arlöv (SE); Rydgren, Göran, S-230 44 Bunkeflostrand (SE); Larsson, Anders, S-244 00 Kävlinge (SE); Brauer, Stefan, S-240 17 Södra Sandby (SE); Linge, Anders, S-244 36 Kävlinge (SE)

(56) References cited:
- EP-A- 0 496 336
- WO-A-92/10228
- WO-A-92/11887
- US-A- 4 547 316
- US-A- 4 905 685
- US-A- 5 099 834

## Description

The present invention relates to a gas mixture intended for delivery, together with a breathing gas, to the lungs of a living being, said gas mixture containing a pre-defined concentration of NO.

The invention also relates to a device for supplying a predetermined amount of NO to the lungs of a living being.

All exchanges between blood gases and the atmosphere take place in the lungs, more particularly in the pulmonary alveoli. When the lungs are defective or diseased, the pulmonary alveoli may be afflicted by dysfunction which impairs or prevents the exchange of gas between the blood and the atmosphere. This is also the case in defects or disease affecting blood vessels in the lungs. Resistance to the flow of blood through the lungs could then increase, thereby increasing the load on the heart. Very small amounts of nitric oxide (NO) can be supplied to the lungs in order to diagnose pulmonary function and treat the lungs. Nitric oxide relaxes smooth muscle in blood vessels and bronchi. Relaxation of the vascular musculature increases the flow of blood through the vessels, thereby improving the exchange of gas between the blood and the atmosphere and reducing the load on the heart. Resistance to flow through the lungs can be determined by measuring the pressure in the pulmonary artery. As a result, the effect of NO administration can be directly measured. If no effect is measurable, the capillaries are either already fully dilated or heavily calcificated. Relaxation of smooth muscle in the bronchi counteracts bronchospasm and asthma. Nitric oxide gas also diffuse through the lung membrane and can be absorbed by the blood in unlimited quantities, in principle, thereby making it possible to measure the diffusion capacity of the lungs by determining the amount of diffused nitric oxide. NO can also be used in different types of respiratory treatment of adults and children and in the monitoring of respiratory treatment and other diagnostic measures.

In WO 92/10228 the use and effects of NO are described in detail. The document also describes how NO in a mixture of N₂ and O₂ can be delivered to a patient's lung with a ventilator.

In EP-A- 0 570 612 is described a device for delivering very small gas flows to a patient. An accurate concentration of e.g. NO can be supplied to a patient's lungs with a very small flow of gas.

Since the amounts of nitric oxide supplied are very small, i.e. generally from one or two ppm up to about 100 ppm, measuring the concentration of nitric oxide supplied to a patient's lungs is difficult. The calculations are especially complicated by the circumstance that known NO meters are sensitive to pressure variations, as occur on the inspiratory side of a ventilator system. In principle, there are four types of meters: a chemoluminance meter which records energy quanta emitted in the chemical reaction NO→NO₂→NO, mass spectrometers, infrared absorption meters and chemical cells which register electron emissions in the reaction NO→ NO₂. In order to measure NO concentrations, the known NO meters also need large, continuous flows of breathing gas. Diverting a large flow from the inspiratory flow before it reaches the patient also complicates flow control.

NO is also a highly reactive gas which chemically reacts with oxygen, O₂, to form nitrogen dioxide, NO₂, a toxic gas. As a rule, relatively large amounts of O₂ are supplied in treatment with NO. To prevent NO from reacting with O₂ before it reaches the lungs, NO must be added to the breathing gas (e.g. air and O₂) close to the patient. This also presents major problems in the measurement of the NO concentration. The gas meter must be placed between the lungs and point at which NO is added. This distance must be as short as possible in order to minimize the amount of NO₂ reaching the lungs. But the amount of NO gas supplied must have time to mix with the breathing gas before the NO concentration is measured. In other words, gas meters will be unable to supply a reliable reading of the NO concentration if NO is added to the breathing gas very close to the patient so as to minimize the formation of NO₂. If NO is added to the breathing gas at a greater distance in order to permit correct measurement of the NO concentration, more NO₂ will form. NO₂ molecules can be filtered out in an absorber arranged before the patient, but the concentration of NO could fall below the desired level if numerous NO₂ molecules are formed. And, as noted above, most modern NO meters require a relatively large, continuous flow of gas past the meter. Any diversion of gases from the patient would give NO time to form more NO₂ molecules before the gas reaches the patient's lungs. NO is generally supplied diluted in N₂, primarily to control the small amounts of NO to be supplied.

One object of the invention is to achieve a gas mixture containing a predetermined concentration of NO so the amount of NO supplied to a patient can be simply and exactly determined.

Another object of the invention is to achieve a device for delivering a predetermined amount of NO to the lungs.

The above-described problems can be solved with a gas mixture in accordance with the invention in that the gas mixture further contains an inert, non-toxic trace gas.

The inert trace gas does not react with the other gases and can be present in a higher concentration than the desired concentration of NO. When NO and trace gas are present in an exact ratio, the concentration of NO can be obtained by measuring the concentration of trace gas in the mixture of gas mixture and breathing gas. The higher concentration of trace gas is easier to measure, and very good accuracy can be achieved. The concentration in gas exhaled from the lungs can also be measured. If the trace gas is absorbed by the body, equilibrium for this uptake must be awaited before the correct measurement values can be obtained when measurement is made of expired gas.

One refinement of the gas mixture is achieved in accordance with the invention in that the inert, non-toxic gas also has limited solubility in water and fat.

A trace gas with limited solubility in water and fat is not absorbed by the body. Equilibrium develops when the lungs achieve the same concentration of trace gas as the concentration supplied, and the concentration of NO can be determined by measuring the concentration of trace gas in inspired gas or in expired gas.

One advantageous trace gas is a noble gas, preferably helium, He. Helium is inert and non-toxic. The gas mixture can therefore consist of NO and He alone. The addition of N₂ to this mixture is not necessary. N2 is otherwise unsuitable as a trace gas, since it is present in the atmosphere.

Since noble gases are also present in well-known concentrations in the atmosphere, and thus in the breathing gas, measurement results must be corrected to compensate for this occurrence.

Another advantageous trace gas is SF₆. SF₆ is an inert gas which does not react with NO, displays minimum solubility in water, is non-toxic and has a high hygienic limit value, 0.5%. Since SF₆ is not absorbed in the body, i.e. it does not pass the lung membrane, it can also be used in conjunction with measures to diagnose pulmonary function, i.e. determination of the lung's functional residual capacity (FRC). FRC can be determined when SF₆ is supplied to the lungs until equilibrium develops between the inspired and expired amounts of SF₆. The administration of SF₆ is then stopped, and the decline in SF₆ concentration in expired air is measured. FRC can then be calculated by determining the volume of SF₆ leaving the lungs. During treatment with NO, FRC can be measured between two different concentration levels for SF₆ in which the lower concentration consists of the therapeutic concentration of NO. In this way, the patient receives the requisite treatment at the same time as FRC is measured.

The concentration of SF₆ in the gas mixture is from 0.1% to 5%, preferably from 1% to 2%. However, the final mixture reaching the patient must never have an SF₆ content greater than the hygienic limit value.

One advantageous form of the gas mixture is obtained in accordance with the invention in that the mixture further contains a predetermined concentration of N₂O.

In contrast to NO, N₂O is only absorbed to a limited degree depending on blood flow. When the body's uptake of N₂O is measured, the rate of blood flow through capillaries in the pulmonary alveoli can be determined.

Irrespective of the gas mixture's total composition of e.g. NO, N₂, He, SF₆, NO or other combinations, it would be advantageous if the concentration of NO were from 10 to 100 000 ppm, preferably from 100 to 10 000 ppm. The concentration supplied to the patient is normally up to 100 ppm, depending on the purpose of the treatment.

A device for supplying a predetermined amount of NO to the lungs is achieved in accordance with the invention in that a device comprising a gas source containing a breathing gas, an inspiration tube, through which breathing gas and NO are fed into the lungs, and an expiration tube, through which breathing gas and NO are led out of the lungs, is characterized in that a second gas source supplies NO to the inspiration tube in the form of a gas mixture according to any of patent claims 1 - 7, and a gas concentration meter is arranged in the gas flowways in order to measure the concentration of trace gas.

By measuring the concentration of trace gas, the concentration of NO is also obtained.

With this set-up, the gas concentration meter can be calibrated after the equipment has been set up but before the patient is hooked up to the device by diverting the flows of breathing gas and gas mixture into the inspiration tube and the expiration tube. If an incorrect gas mixture is connected by mistake, e.g. a mixture with 10 000 ppm NO and 2% SF₆ instead of 1 000 ppm NO and 1% SF₆, the measurement error, even with a uncalibrated gas concentration meter, will be so large that it will be obvious to the operator that an incorrect gas mixture gas been connected.

It would be advantageous if the gas concentration meter were arranged in the expiration tube in order to measure the concentration of trace gas. Since the trace gas achieves equilibrium in the body or is not absorbed by the body at all, measurement can be made of expired gas and still supply exact information on the concentration supplied. Moreover, the gas mixture here can be delivered, in principle, directly to the lungs, since no meter is needed on the inspiratory side. The formation of NO₂ is hereby minimized. A refinement of the device is achieved in accordance with the invention in that the device further comprises a flow meter in the expiration tube to measure the flow of expired gas and an analyzer to determine functional residual capacity, FRC, whereby the analyzer controls the second gas source so it increases the amount of gas mixture added during a flushing-in phase from a first level to a second level until the trace gas attains equilibrium in the lungs, whereupon the analyzer controls the second gas source so it, at the start of a flushing-out phase, again adds gas mixture on the first level and, on the basis of gas concentration measurements made by the gas concentration meter and flow measurements made by the flow meter during the flushing-out phase of the elevated trace gas concentration, calculates FRC.

In this manner, any ongoing treatment with NO does not have to be terminated for calculation of FRC. Instead, FRC is determined between two different levels of trace gas concentration.

It would be advantageous if the device were devised so a first additional gas concentration meter were arranged in the expiration tube in order to measure the concentration of NO.

Measurement of the trace gas makes the amount of supplied NO known, and NO uptake in the body can be determined when the amount of NO exhaled is measured, thereby making possible determination of the lung's diffusion capacity. In principle, the entire gas flow can be used here for measuring the concentration of residual NO.

In conjunction with delivery to the lungs of a gas mixture, which also contains a predetermined concentration of N₂O, it would be advantageous if a second additional gas concentration meter were arranged in the expiration tube to measure the concentration of N₂O. As is the case with NO, this would yield the concentration of inspired gas from measurement of the trace gas. Uptake in the body can therefore be easily calculated.

In a simultaneously submitted patent application with publication no. EP-A-640 356 is described an apparatus which utilizes another principle for delivering e.g. NO to a lung.

A refinement of the device is achieved in accordance with the invention in that an expiration valve is arranged in the expiration tube between the lungs of the living being and the gas concentration meter(s).

The expiration valve regulates pressure in the expiration tube during both inspiration and expiration.

Arrangement of the expiration valve in the expiration tube between the lungs and gas concentration meter(s) also makes possible to lead the inspiratory flow undisturbed to the patient.

It would be advantageous if the device were devised so a mixing container were arranged on the expiration tube between the expiration valve and the gas concentration meter(s).

Especially when slow gas concentration meters are used, measuring a floating average for the gas concentration, instead of a momentary value, would be advantageous. A corresponding average value for flow should also be determined at the same time. One minute is a suitable period for averaging. The mixing container is important in this instance, since it forms an average concentration for the gas passing through it. On the average, gas passes in a certain amount of time during which gases from a longer period of time intermix, i.e. forms an average value for the average time through the container.

The problems in the formation of NO₂ have been mentioned above. Since this gas continuously forms at a specific reaction rate, determination of the NO concentration can be compensated for the formation of NO₂. When the concentration of NO₂ is measured on the expiratory side, the determination of the concentration of inspired NO and the diffusion of NO in the body can be compensated. A third possibility is to calibrate the entire system with a known gas mixture before the patient is hooked up to the device. Measurement of the NO₂ concentration can also be used to check for any leaks on the high-pressure side to ensure there is an influx of O₂. This monitoring can be connected to e.g. an alarm.

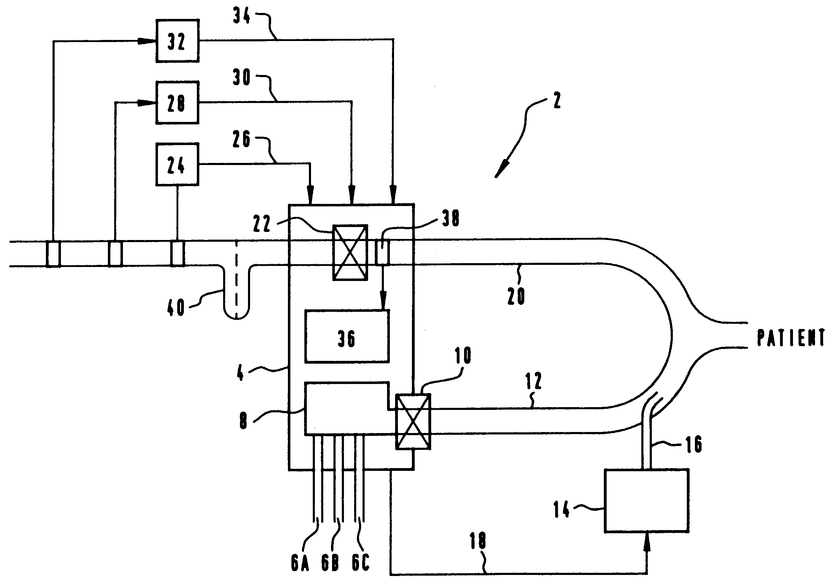

Referring to the figure, one embodiment of the device according to the invention will be described below.

A device for delivering a predetermined concentration of NO to the lungs of a patient is generally designated 2 in the figure. The device comprises a ventilator unit 4 which controls and regulates the entire device 2. Three gas connectors 6A, 6B, 6C are connected to a gas mixer 8 in the ventilator unit 4. The gases constituting a breathing gas for the patient are fed to the ventilator unit 4 through the gas connectors 6A, 6B, 6C. These gases could be e.g. oxygen and air, two of the connectors 6A, 6B, 6C then being used. From the gas mixer 8, breathing gas passes via a first valve 10 at a predetermined pressure and flow to an inspiration tube 12. The inspiration tube 12 leads the breathing gas to the patient's lungs. From a second gas mixer 14, a gas mixture containing a predetermined concentration of NO, SF₆ and N₂O, diluted with N₂ or He, can be fed to the inspiration tube 12 via a connecting tube 16. The ventilator unit 4 controls the gas mixture supplied via a control line 18. NO is strongly reactive and forms, with oxygen, O₂, nitrogen dioxide, NO₂, a toxic gas. So gas mixture should be added as close to the patient as possible to minimize the concentration of NO₂ and to ensure that the desired concentration of NO is delivered to the lungs. The gas mixture could contain e.g. 0.1% NO, 2% SF₆, 1% N₂O, N₂ or He making up the rest.

Gas exhaled by the patient passes through an expiration tube 20 back to the ventilator unit 4. This expired gas is then led away via a second valve 22 to evacuation, e.g. into ambient air or collection vessels for expired gas. A positive endexpiratory pressure (PEEP), for example, can be maintained with the second valve 22.

In the expiration tube 20, after the second valve 22, there is a first gas meter 24 which measures the concentration of SF₆ in expired gas and sends a measurement signal, via a first signal line 26, to the ventilator unit 4, a second gas meter 28, which measures the concentration of NO in expired gas and sends a measurement signal, via a second signal line 30, to the ventilator unit 4, and a third gas meter 32, which measures the concentration of N₂O in expired gas and sends a measurement signal, via signal line 34, to the ventilator unit 4.

A mixing container 40 is arranged between the gas meters 24, 28, 32 and the second valve 22. The mixing container 40 makes possible collection and mixing of gas over a long period of time, thereby forming an average value for the concentration of NO, N₂O and N₂. SF₆ is unaffected.

The addition of NO via the second gas mixer 14 can be performed for diagnostic or therapeutic reasons, e.g. for determining the diffusion capacity of the lung or for reducing the resistance to blood perfusion in the pulmonary alveoli. The known ratio between the concentration of NO and SF₆ in the gas mixture from the second mixer 14 makes it possible to determine the amount of NO delivered to the patient by measurement of the concentration of SF₆ in the expiration tube 20. SF₆ is an inert gas which is not absorbed in the body, and equilibrium develops between inspired and expired SF₆ after a small number of respiratory cycles. If the concentration of NO in the mixed inspiratory gas changes, the concentration of SF₆ also changes, and a new equilibrium concentration will develop in the lungs, as measured in the expired gas. In this manner, the amount of NO supplied can be determined exactly. Compensation for the NO₂ which still forms can be made in any of the ways described above.

The concentration of trace gas can also be measured in the inspiration tube. The equilibrium concentration of the lungs' content of trace gas will then not affect the measurement, and all changes are recorded immediately. However, measurement on the expiratory side conveys other advantages. The inspiratory flow can pass undisturbed to the lungs. The gas mixture can be added even closer to the lungs, in principle inside the lungs, thereby minimizing reactions between NO and O₂. Breathing gas and the gas mixture also have time to mix thoroughly before measurement takes place, something more difficult to achieve on the inspiratory side when the gases are to be mixed very close to the lungs.

When the concentration of NO in expired gas is measured. the amount of NO absorbed by the body can be determined. In this way, the diffusion capacity of the lungs can be determined, since NO molecules can, in principle, be absorbed by blood in unlimited amounts, so no counter-pressure builds up to limit diffusion of NO into the blood.

When N₂O, whose concentration is obtained in the same way as in determination of NO, i.e. through measurement of the concentration of SF₆, is added and the N₂O content of expired gas is measured, the flow of blood through the lungs can be determined. This is because N₂O molecules, in contrast to NO molecules, can only be absorbed into blood to a limited degree. The uptake of N₂O molecules is therefore a measure of blood perfusion through the lungs. N₂O can be advantageously added in two different concentrations, and the change in the concentration of N₂O in expired gas can then be measured to determine the flow of blood through the lungs.

In the device 2 there is also an analyzer 36 for determining functional residual capacity, FRC. The analyzer 36 can control the second gas source 14 and receive measurement values from the gas concentration meters 24, 28, 32 and from a flow meter 38 which measures the flow of expired gas. When the flow from the second gas source 14 is increased for a given period of time, a new equilibrium develops for the SF₆ concentration in the lungs. When the flow from the second gas source 14 is restored, the lungs will gradually be purged of surplus SF₆. The analyzer can calculate the volume of this surplus on the basis of concentration values and flow values measured in the time required to flush surplus SF₆ out of the lungs. The calculated volume is then used for determining FRC.

Above it was noted that only two of the three gas connectors 6A, 6B, 6C are used when air and O₂ serve as the breathing gas. In certain situations, the addition of gas mixture through the third gas connector 6C may then be appropriate instead of having a separate connector in the inspiration tube 12. The first valve 10 can be replaced with three valves, each of which controlling the flow of gas from the three gas connectors 6A, 6B, 6C. In this version, a Servo Ventilator 300 from Siemens-Elema, Sweden, can be advantageously used.

## Claims

1. A gas mixture intended for delivery, together with a breathing gas, to the lungs of a living being, said gas mixture containing a predetermined concentration of NO, **characterized in** that the gas mixture contains a predetermined concentration of an inert, non-toxic trace gas.

2. A gas mixture according to claim 1, **characterized in** that the inert, non-toxic trace gas only displays poor solubility in water and fat.

3. A gas mixture according to claim 1, **characterized in** that the trace gas is a noble gas, preferably helium, He.

4. A gas mixture according to claim 2, **characterized in** that the trace gas is SF₆.

5. A gas mixture according to claim 4, **characterized in** that the concentration of SF₆ ranges from 0.1 to 5%, preferably from 1 to 2%.

6. A gas mixture according to any of the above claims, **characterized in** that the gas mixture further contains a predetermined concentration of N₂O.

7. A gas mixture according to any of the above claims, **characterized in** that the concentration of NO is from 10 to 100 000 ppm, preferably from 100 to 10 000 ppm.

8. Device for delivering a predetermined amount of NO to the lungs of a living being, comprising a gas source (4) containing a breathing gas, an inspiration tube (12), through which breathing gas and NO can be fed into the lungs, and an expiration tube (20), through which breathing gas and NO can be led out of the lungs**, characterized in** that a second gas source (14) is arranged to supply NO to the inspiration tube (12) in the form of a gas mixture according to any of patent claims 1 - 7, and that a gas concentration meter (24) is arranged in the gas flowways in order to measure the concentration of trace gas.

9. A device according to claim 8, **characterized in** that the gas concentration meter (24) is arranged in the expiration tube (20) to measure the concentration of trace gas.

10. A device according to claim 9, **characterized by** a flow meter (38), arranged in the expiration tube (20) to measure the flow of expired gas, and an analyzer (36) to determine functional residual capacity, FRC, whereby the analyzer (36) controls the second gas source (14) to increase the amount of gas mixture added during a flushing-in phase from a first level to a second level until the trace gas as measured by the gas concentration meter (24) attains equilibrium in the lungs, whereupon the analyzer (36) controls the second gas source (14) to, at the start of a flushing-out phase, again add gas mixture on the first level and, on the basis of gas concentration measurements made by the gas concentration meter (24) and flow measurements made by the flow meter (38) during the flushing-out phase of the elevated trace gas concentration, calculate FRC.

11. A device according to any of claims 8 - 10, **characterized by** a first additional gas concentration meter (28), arranged in the expiration tube (20), to measure the concentration of NO.

12. A device according to claims 8 - 11, **characterized in** that the second gas source (14) adds a gas mixture according to claim 6 to the inspiration tube (12), and a second additional gas concentration meter (32) is arranged in the expiration tube (20) in order to measure the concentration of N₂O.

13. A device according to any of claims 8 - 12, **characterized by** an expiration valve (22), arranged in the expiration tube (20) between the lungs of the living being and the gas concentration meter(s) (24, 28, 32).

14. A device of claim 13, **characterized by** a mixing container (40), arranged in the expiration tube (20) between the expiration valve (22) and the gas concentration meter(s) (24, 28, 32).

## Patentansprüche

1. Eine Gasmischung, die zur Zuführung, zusammen mit einem Atemgas, zu den Lungen eines Lebewesens vorgesehen ist, wobei die Gasmischung eine vorbestimmte Konzentration von NO enthält, **dadurch gekennzeichnet**, **daß** die Gasmischung eine vorbestimmte Konzentration eines inerten, nichttoxischen Spurengases enthält.

2. Eine Gasmischung nach Anspruch 1, **dadurch gekennzeichnet**, daß das inerte, nichttoxische Gas eine schlecht Löslichkeit in Wasser und Fett aufweist.

3. Eine Gasmischung nach Anspruch 1, **dadurch gekennzeichnet**, daß das Spurengas ein Edelgas, vorzugsweise Heliüm, He, ist.

4. Eine Gasmischung nach Anspruch 2, **dadurch gekennzeichnet**, daß das Spurengas SF₆ ist.

5. Eine Gasmischung nach Anspruch 4, **dadurch gekennzeichnet**, daß die Konzentration von SF₆ in dem Bereich von 0.1 bis 5%, vorzugsweise von 1 bis 2% liegt.

6. Eine Gasmischung nach einem der obigen Ansprüche, **dadurch gekennzeichnet**, daß die Gasmischung zusätzlich eine vorbestimmte Konzentration von N₂O enthält.

7. Eine Gasmischung nach einem der obigen Ansprüche, **dadurch gekennzeichnet**, daß die Konzentration von NO zwischen 10 und 100 000 ppm, vorzugsweise zwischen 100 und 10 000 ppm liegt.

8. Vorrichtung zur Zuführung einer vorbestimmten Menge von NO zu den Lungen eines Lebewesens mit einer Atemgas enthaltenden Gasquelle (4), einer Inspirationsleitung (12), über die Atemgas und NO den Lungen zugeführt werden kann, und einer Exspirationsleitung (20), über die Atemgas und NO aus den Lungen herausgeführt werden kann, **dadurch gekennzeichnet**, daß eine zweite Gasquelle (14) angeordnet ist, um der Inspirationsleitung (12) NO in der Form einer Gasmischung gemäß irgend einem der Patentansprüche 1 - 7 zuzuführen, und daß ein Gaskonzentrationsmesser (24) in den Gasflußwegen angeordnet ist, um die Konzentration des Spurengases zu messen.

9. Eine Vorrichtung nach Anspruch 8, **dadurch gekennzeichnet**, daß der Gaskonzentrationsmesser (24) in der Exspirationsleitung (20) zum Messer der Konzentration des Spurengases angeordnet ist.

10. Eine Vorrichtung nach Anspruch 9, **gekennzeichnet durch** einen zum Messen des Exspirationsgasflusses in dem Exspirationsschlauch (20) angeordneten Flußmesser (38) und einen Analysator (36) zum Bestimmen der funktionalen Restkapazität, FRC, wobei der Analysator (36) die zweite Gasquelle (14) steuert, um die Menge der Gasmischung, die während der Ausspülphase hinzugefügt wird, von einem ersten Niveau auf ein zweites Niveau zu erhöhen, bis das Spurengas, dasdurch den Gaskonzentrationsmesser (24) gemessen wird, ein Gleichgewicht in den Lungen erreicht, woraufhin der Analysator (36) die zweite Gasquelle (14) steuert, um zu Beginn der Ausspülphase erneut eine Gasmischung mit dem ersten Niveau hinzuzufügen und auf der Basis der von dem Gaskonzentrationsmesser (24) durchgeführten Gaskonzentrationsmessungen und von dem Flußmesser (38) durchgeführten Flußmessungen während der Ausspülphase der erhöhten Spurengaskonzentration FRC zu berechnet.

11. Eine Vorrichtung nach einem der Ansprüche 8 - 10, **gekennzeichnet durch** einen zusätzlichen Gaskonzentrationsmesser (28), der zum Messen der Konzentration von NO in dem Exspirationsschlauch (20) angeordnet ist.

12. Eine Vorrichtung nach den Ansprüchen 8 - 11**,dadurch gekennzeichnet**, daß die zweite Gasquelle (14) eine Gasmischung gemäß Anspruch 6 dem Inspirationsschlauch (12) zufügt und ein zweiter zusätzlicher Gaskonzentrationsmesser (32) zum Messen der Konzentration von N₂O in dem Exspirationsschlauch (20) angeordnet ist.

13. Eine Vorrichtung nach einem der Ansprüche 8 - 12, **gekennzeichnet** durch ein Exspirationsventil (22), das in dem Exspirationsschlauch (20) zwischen den Lungen des Lebewesens und dem (den) Gaskonzentrationsmesser(n) (24, 28, 32) angeordnet ist.

14. Eine Vorrichtung nach Anspruch 13, **gekennzeichnet durch** einen Mischbehälter (40), der in dem Exspirationsschlauch (20) zwischen dem Exspirationsventil (22) und dem (den) Gaskonzentrationsmesser(n) (24, 28, 32) angeordnet ist.

## Revendications

1. Mélange gazeux destiné à être fourni, ensemble avec un gaz de respiration, aux poumons d'un être vivant, le mélange gazeux contenant une concentration déterminée à l'avance de NO, caractérisé en ce que le mélange gazeux contient une concentration déterminée à l'avance d'un gaz inerte, non toxique, à l'état de trace.

2. Mélange gazeux suivant la revendication 1, caractérisé en ce que le gaz inerte, non toxique, à l'état de trace présente uniquement une solubilité médiocre dans l'eau et les graisses.

3. Mélange gazeux suivant la revendication 1, caractérisé en ce que le gaz à l'état de trace est un gaz noble, de préférence l'hélium, He.

4. Mélange gazeux suivant la revendication 2, caractérisé en ce que le gaz à l'état de trace est du SF₆.

5. Mélange gazeux suivant la revendication 4, caractérisé en ce que la concentration de SF₆ est comprise entre 0,1 et 5%, de préférence entre 1 et 2%.

6. Mélange gazeux suivant l'une quelconque des revendications précédentes, caractérisé en ce que le mélange gazeux contient en outre une concentration déterminée à l'avance de N₂O.

7. Mélange gazeux suivant l'une quelconque des revendications précédentes, caractérisé en ce que la concentration de NO est comprise entre 10 et 100 000 ppm, de préférence entre 100 et 10 000 ppm.

8. Dispositif pour fournir une quantité déterminée à l'avance de NO aux poumons d'un être vivant, comportant une source (4) de gaz comportant un gaz de respiration, un tube (12) d'inspiration, par lequel du gaz de respiration et du NO peuvent être envoyés dans les poumons, et un tube (20) d'expiration, par lequel du gaz de respiration et du NO peuvent être évacués des poumons, caractérisé en ce qu'une seconde source (14) de gaz est disposée pour fournir du NO au tube (12) d'inspiration sous la forme d'un mélange gazeux, suivant l'une quelconque des revendications 1 à 7 du brevet, et en ce qu'un dispositif (24) de mesure de concentration de gaz est disposé dans les voies d'écoulement de gaz afin de mesurer la concentration du gaz à l'état de trace.

9. Dispositif suivant la revendication 8, caractérisé en ce que le dispositif (24) de mesure de concentration en gaz est disposé dans le tube (20) d'expiration pour mesurer la concentration en gaz à l'état de trace.

10. Dispositif suivant la revendication 9, caractérisé par un dispositif (38) de mesure de débit, disposé dans le tube (20) d'expiration pour mesurer le débit de gaz expiré, et un dispositif (36) d'analyse destiné à déterminer la capacité résiduelle fonctionnelle, FRC, grâce à quoi le dispositif (36) d'analyse commande la seconde source (14) de gaz de manière à augmenter la quantité de mélange gazeux ajoutée pendant une phase de balayage vers l'intérieur d'un premier niveau jusqu'à un second niveau jusqu'à ce que le gaz à l'état de trace, tel que mesuré dans le dispositif (24) de mesure de concentration de gaz, atteigne un équilibre dans les poumons, ensuite le dispositif (36) d'analyse commande la seconde source (14) de gaz pour, au début d'une phase de balayage vers l'extérieur, de nouveau ajouter du mélange gazeux au premier niveau, et, sur la base des mesures de concentration de gaz réalisées par le dispositif (24) de mesure de concentration de gaz et des mesures de débit réalisées par le dispositif (38) de mesure de débit pendant la phase de balayage vers l'extérieur de la concentration de gaz à l'état de trace augmenté, calculer la FRC.

11. Dispositif suivant l'une quelconque des revendications 8 à 10, caractérisé par un premier dispositif (28) de mesure en concentration de gaz supplémentaire disposé dans le tube (20) d'expiration, pour mesurer la concentration de NO.

12. Dispositif suivant l'une quelconque des revendications 8 à 11, caractérisé en ce que la seconde source (14) de gaz ajoute un mélange gazeux suivant la revendication 6 au tube (12) d'inspiration, et un second dispositif (32) de mesure de concentration de gaz supplémentaire est disposé dans le tube (20) d'expiration afin de mesurer la concentration de N₂O.

13. Dispositif suivant l'une quelconque des revendications 8 à 12, caractérisé par une vanne (22) d'expiration, disposée dans le tube (20) d'expiration entre les poumons de l'être vivant et le ou les dispositif(s) de mesure (24, 28, 32) de concentration de gaz.

14. Dispositif suivant la revendication 13, caractérisé par un conteneur (40) de mélange, disposé dans le tube (20) d'expiration entre la vanne (22) d'expiration et le ou les dispositif(s) de mesure (24, 28, 32) de concentration de gaz.
